# EUROPEAN PATENT APPLICATION

(11) **EP 3 674 915 A1**
(43) Date of publication of application: **01.07.2020**
(21) Application number: 18382996.9
(22) Date of filing: 27.12.2018
(51) Int. Cl.: G06F 16/2457, G06N 5/04, G06F 16/908

(54) **METHOD AND SYSTEM FOR AUTOMATIC OPTIMIZATION OF USER'S BEHAVIOURAL CHANGES**

(71) Applicant: Telefonica Innovacion Alpha S.L, 28050 Madrid (ES)
(72) Inventor: HARRISON, Oliver, 28050 MADRID (ES); MATIC, Aleksandar, 28050 MADRID (ES)
(74) Representative: Herrero & Asociados, S.L.

(57) **Abstract**

A system and method for optimizing user's behavioural changes comprising:
- for a time instant, t, adquiring static user characteristics (101) to generate a user characteristics vector (110) and dynamic user characteristics which define a current user state (102) to generate a user current state vector (120),
- querying (103) an artificial intelligence system (400) using the generated vectors (110, 120) as input to generate as output (104) a behavioural change tool vector (130) for the time instant, t, delivered to the user who makes selections to change behaviour based on the output (104);
- mapping the vectors (110, 120, 130) generated for the time instant, t, to a next current state vector (120) for a next time instant, t+1;
- repeating the querying (103) to the artificial intelligence system (400) using as input the three vectors updated over time, until the behavioural change goal is achieved; the selections taken by the user defining an optimal path for behavioural changes.

## Description

### Field of the invention

The present invention has its application within the telecommunication sector, more specifically, relates to the deployment of tools in telecommunication network entities (e.g., applications in mobile user terminals such as smartphones or tablets or for wearable programmable electronic devices, computer programs, etc.) which work systematically with a user for improving his/her personal motivation for well-being.

More particularly, the present invention refers to a system and method for optimizing behavioural changes of an individual.

### Background of the invention

Since 2008, the leading cause of death in the United States has become personal choices and the same trend is seen in many countries worldwide. In this context, behaviour change programmes which address such personal choices have the potential to deliver considerable benefits to both the general health of the population and to the cost burden of disease across the population. For many people changing health-related behaviours is very challenging. For this reason, despite decades of health education, smoking rates have plateaued, and obesity rates are rising worldwide.

To achieve positive impact on health and well-being, behaviour change services may deploy a range of approaches and typically require lots of time.

US 2010/153287 A1 discloses a system and method that guides patients, caregivers and supporters or individuals as they confront, and endeavor to overcome, a serious health crisis or major life transition. The system facilitates the reduction of the overwhelming psychological nature of the diagnosis event by compartmentalizing it into manageable phases, based on distance from diagnosis, enabling a patient to overcome and quickly envision their first step toward wellbeing.

US 2018/068573 A1 discloses a method for influencing a user's perception of a subject. The method obtains a desired value associated with a sensory trigger relevant to the user's perception and is configured to target an automatic mind of the user. Via a feedback interface, a subconscious response is stimulated to influence the user's perception.

US 2014/323817 A1 discloses a computer-implemented method for mental state analysis by correlating mental state information of an individual with mental state information from a plurality of people and categorizing the individual with the others based on the correlation.

WO 2017/176653 A1 discloses the generation of predictive models to suggest beneficial actions based on user's data over time. The system delivers the suggested action or circumstance to a device carried by or worn by a user in the form of advice or a story, using text, speech, images, or video.

US 2010/037170 A1 discloses a system configured to provide to a graphical user interface enabling a user to establish and monitor progress towards a personal objective, and receive data input from the user.

One reason for the limited impact of behaviour change programmes to date is that advice from healthcare professionals and public health campaigns is not personalised for each user. In addition, for people embarking on behaviour change programmes the extent of the change (e.g., moving from being a smoker of 60-a-day to becoming a non-smoker, or losing 20% of body weight) often creates an overwhelming sense of the challenge, and thus a significant psychological barrier to both getting started and making positive progress.

There is a myriad of behavioural change theories, however it is, today, unclear how to personalise the behavioural change approach to an individual for an improved effectiveness.

Therefore, there is a need in the state of the art for developing a system that identifies an optimal path bulit based on a personalised selection of behaviour change approaches to optimise the effectiveness of behaviour change.

### Summary of the invention

The present invention solves the aforementioned problems and overcomes previously explained state-of-art work limitations by providing a system and a method to identify an optimal path for an individual user at a specific time based on a model that combines user characteristics, user preferences, and data from other users. The invention provides a tool to personalise the selection of behaviour change approaches and compartmentalise the overall behaviour change programme into a personalised set of more manageable steps. Both the approach selection and the concatenation of steps are derived from a mathematical model that combines user characteristics, user preferences, and data from other users (matched for relevance to the proband user). Thus the identified optimal path comprises both the defined steps in which specific approaches are deployed, and the sequence of those steps over time. The aim is to enable the user to quickly envision simple first steps toward well-being and maintain personal motivation across each step through the positive psychological impact of successfully completing interim steps.

In the context of the invention, an "optimal path" is the selection of approaches and the concatenation of steps defined for each user, through a vector space comprising potential behaviour change approaches delivered over time, the sequence of steps constituting a behavioural change programme at an individual level. The vector space comprises current and target user behaviour, potential behaviour change approaches and sequencing of individual steps over time. The optimal path is defined by dynamically setting each step in the behavioural change programme through an artificial intelligence system built on a database containing the vector space. The artificial intelligence system takes as input parameters: user characteristics, user states and the information about the administered behavioural change tool over time.

In order to select the steps that define an optimal path, critical user characteristics are identified, the user characteristics including:
∘ Semi-static or static characteristics (e.g., risk appetite, time-discounting, biological gender, Big-5 Personality), and
∘ Dynamic characteristics (e.g., mood, momentary happiness, boredom states)

In order to identify the critical user characteristics tools derived from Personal Construct Theory (e.g., manual/automated Repertory Grids) which enable the quantitative estimation of positive and negative associations are used. In addition, an analysis of empirical data from previous trials for other users (in which behaviour change approaches were matched to users and the effectiveness of those approaches was quantified) is performed. Furthermore, continuous adaptation of the aforementioned analysis is carried out based on reinforcement learning or other existing approaches.

Finally, the optimal sequencing of individual approaches for each step is mapped into an overall behaviour change programme (concatenation).

A first aspect of the present invention refers to a computer-implemented method for optimizing behavioural changes of a user, having a behavioural change goal set for the user, which, for a specific time instant, t, adquires static user characteristics to generate a user characteristics vector and dynamic user characteristics to generate a user current state vector. The method allows the query to an artificial intelligence system using as input the generated user characteristics and user current state vectors. The method generates by means of artificial intelligence a behavioural change tool vector for the specific time instant, t, delivered as output to the user, who makes one or more selections to change behaviour based on this output behavioural change tool. The method maps the user characteristics vector, user current state vector and behavioural change tool vector generated for the specific time instant, t, to a next current state vector generated for a next time instant, t+1; and repeats the query to the artificial intelligence system using as input the three vectors updated over time until the mapping indicates that the behavioural change goal is achieved; the selections taken by the user defining an optimal path for behavioural changes.

A second aspect of the present invention refers to a system configured to implement the method described before by means of artificial intelligence built on a database, the artificial intelligence processing means and the database forming an artificial intelligence system. The artificial intelligence system delivers the behavioural change tool vector as an output to the user, and uses as input the generated user characteristics vector and user current state vector (and behavioural change tool vector), stored in the database and updated over time.

The method and system in accordance with the above described aspects of the invention has a number of advantages with respect to the aforementioned prior art, which can be summarized as follows:
- Greater effectiveness: The invention helps reduce the psychological barrier to embarking upon, and achieving, behaviour change by compartmentalizing the overall vector into steps over time, where each step is selected for likelihood of both efficacy and engagement for that user at that time; for each step, the system identifies the most effective approach, and the system identifies a sequence of steps that will maximise effectiveness over time. By tailoring approaches and tools to individual user characteristics, optimal paths increase user uptake, adherence to a health and well-being improvement programme, and the efficacy of individual approaches and tools, and the overall health and well-being improvement programme.
- Continuous improvement: Optimal path calculations are continuously updated based on empirical evidence of the effectiveness of approaches, tools and programmes. Through a reinforcement learning mechanism, optimal paths are refined to maximise their effectiveness. Such learnings are incorporated into the optimal path service on a real-time basis and available to subsequent users.
- Cost saving: Since optimal path calculations are automated, they represent a lower-cost approach to delivering the functionality than human or manual approaches existing in the prior art.

These and other advantages will be apparent in the light of the detailed description of the invention.

### Description of the drawings

For the purpose of aiding the understanding of the characteristics of the invention, according to a preferred practical embodiment thereof and in order to complement this description, the following Figures are attached as an integral part thereof, having an illustrative and non-limiting character:
Figure 1 shows a flow diagram of a method for optimizing the behavioural changes of a user, according to a possible embodiment of the invention.
Figure 2 shows a schematic block diagram of a system for adquiring user state and user characteristics to be used in defining an optimal path for the user's behavioural changes, according to a possible embodiment of the invention.
Figure 3 shows an artificial intelligence system for setting the steps of the optimal path for the behavioural changes based on the adquired user state and characteristics, according to a possible embodiment of the invention.

### Preferred embodiment of the invention

The matters defined in this detailed description are provided to assist in a comprehensive understanding of the invention. Accordingly, those of ordinary skill in the art will recognize that variation changes and modifications of the embodiments described herein can be made without departing from the scope and spirit of the invention. Also, description of well-known functions and elements are omitted for clarity and conciseness.

Of course, the embodiments of the invention can be implemented in a variety of architectural platforms, operating and server systems, devices, systems, or applications. Any particular architectural layout or implementation presented herein is provided for purposes of illustration and comprehension only and is not intended to limit aspects of the invention.

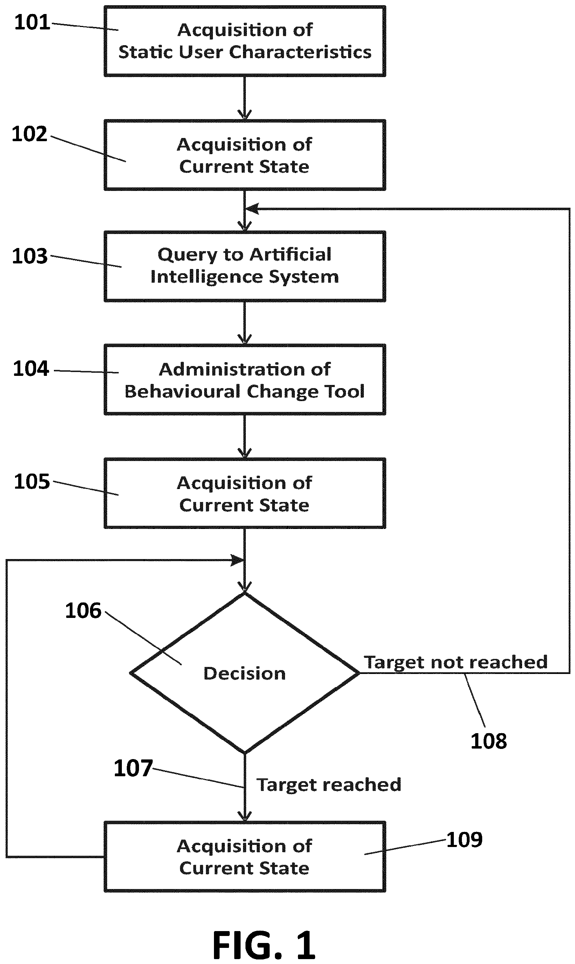

Figure 1 shows a flow diagram of a method for optimizing the user's behavioural changes. Given a behavioural change goal, the method starts with the acquisition of a set of personal static user characteristics (101) and the current state (102) of a user, fed as inputs into an artificial intelligence system (400) as a query (103), which outputs (104) the recommended behavioural change tool with its features. The user's current state is updated (105) before deciding (106) whether the behavioural change goal is reached (107) or not (108). The user's current state is adquired (109) over time to ensure that the behavioural change goal is reached (107). When the goal is not achieved (108), the query (103) to the artificial intelligence system (400) is repeated in order to get an adjusted behavioural change tool/programme.

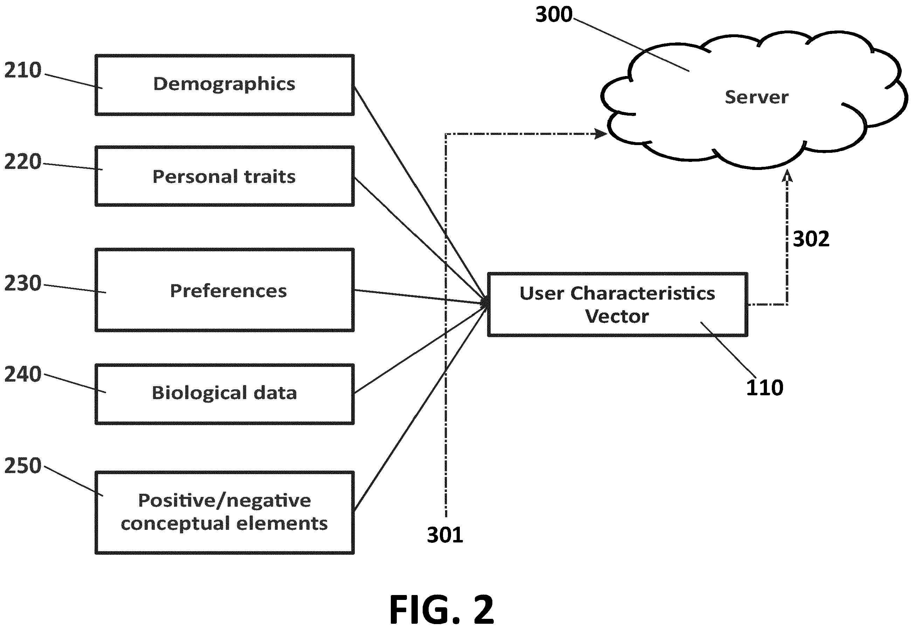

The user characteristics vector (110), shown in Figure 2, is an input to the Artificial Intelligence System (400) and comprises:
- demographic information (210), e.g. gender, age, ethnic group, religion, socio-economic status, relationship status, etc.;
- personal traits (220), e.g. BIG-5, boredom proneness, or even cognitive abilities such as IQ);
- preferences (230) including a wide range of personal descriptors, e.g. the inclination towards specific products, items, colours, hobbies, topics, ideas, etc;
- biological characteristics (240), e.g., genetics, physiological measurements; and
- positive/negative conceptual associations (250), defining object associations in the domain space of the targeted behavioural change (e.g., in case of dietary goals, the associations may include links between specific food categories, feelings after eating, obesity, well-being, drinks...).

The source of demographics (210), personal traits (220), preferences (230), biological data (240), positive/negative conceptual elements (250), can include direct inputs from a user, and/or external data bases, and/or external services, and/or from sensor data.

In one embodiment, all these data are sent (301) to a server (300) which generates the user characteristics vector (110) based on the received data.

In another embodiment, the user characteristics vector (110) is computed locally, at the client side, by extracting relevant features that may be anonymous and then this vector (110) is sent (302) to a server (300).

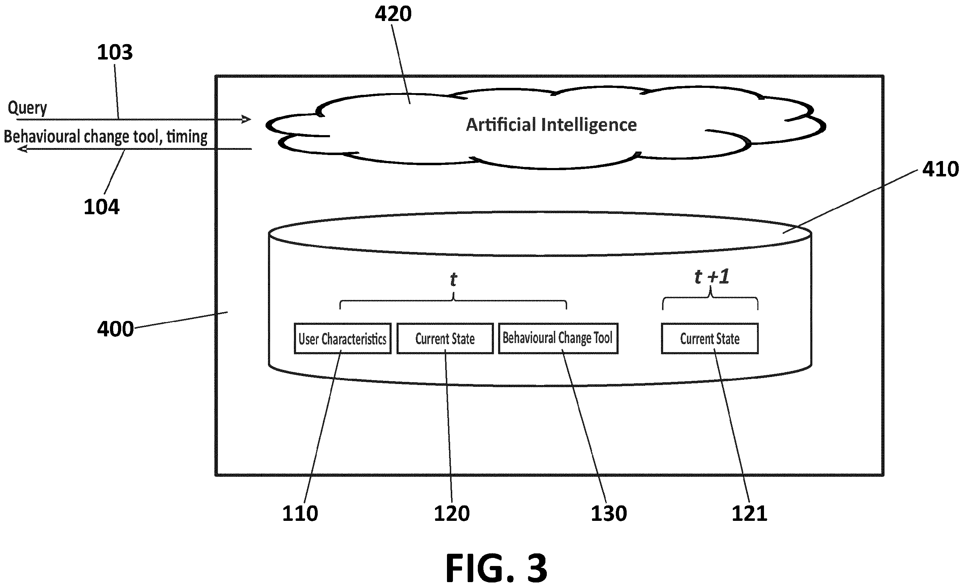

The user state vector (120), shown in Figure 3, is defined as a dynamically changing vector constituted of three sub-vectors:
- Current behaviours. The sub-vector of current behaviours represents the behavioural domain of the targeted behavioural change, but it may also include the patterns of activities of daily living (such as sleep, diet, sports, social activities, whereabouts,...).
- Current (dynamic) personal characteristics. The sub-vector of dynamic personal characteristics refers to momentary cognitive processing, such as risk appetite, executive cognitive functions, time discounting, etc.
- Emotional status. The sub-vector of emotional status includes the assessment of user's emotional and wellbeing indices.

The three sub-vectors can be acquired using internal or external services that rely on personal devices (e.g. mobile phones, tablets, computers, wearables that measure physical or physiological signals,...), ambient sensors (e.g. presence sensor, video/audio, temperature, air monitors, etc.), and direct user inputs (through questionnaires, surveys, notifications).

The Behavioural Change Tool or Programme can be defined as a behavioural change tool vector (130), shown in Figure 3, which comprises a start (trigger), content and an end. The behavioural change tool vector (130) is delivered (104) by the Artificial Intelligence System (400) to a user as an action to be performed from, for instance, the following (but not conclusive) set of action types:
- GENERIC TIPS e.g. "Go to bed each day at the same time makes you rest better"
- MOTIVATIONAL e.g. Buddha, Einstein quotes, Thought of the day
- PERSONAL TIP e.g. "To make use of your time during your commute, try TED Talks."
- MINI GOAL e.g. "Sleep better than your mum this week", "Take a different route now"
- POINT OF DECISION e.g. Personalised prompts, rules, feedback within 3rd party experience (bank, supermarket)
- EDUCATION e.g. Education cards, Mini surveys, Videos, Audio guides
- CHAT ACTIONS e.g. Book a table using OpenTable, Find a different route to work on Google Maps..
- GAMES e.g. Attention training, Cognitive test games, Anti Bias balloons
- EXERCISE e.g. Exposure exercise, Rethink your thoughts.
- CONVERSATION e.g. TLC Conversations on stress, can include educational cards, chat actions and games.
- OTHER APP e.g. "Why do not use Strava for running. Click here"
- PROGRAM, e.g. Run 20 km in 10 days, CBT for BDD (Mix of other tools)
- NOTIFICATION e.g. "Tell us how you are feeling", "Priming messages"
- BUDDY e.g. Get motivation by helping others or get help your self.
- EXPERT e.g. Therapist, Financial advisor, Gym coach

The Artificial Intelligence System (400) comprises an Artificial Intelligence (410) built on a database (420) which contains the aforementioned vectors, shown in Figure 3, user characteristics vector (110), user current state vector (120) and behavioural change tool vector (130), the three vectors (110, 120, 130) being referred to a specific time instant t and mapped to the next current state vector (121) which is measured at a specific next time instant t+1. In one embodiment, the Artificial Intelligence (410) is based on the reinforcement learning, where the state space is represented with a subset of elements in the current state vector (120), context is represented with a subset of elements in the current state vector (120) and personal characteristics vector (110) measured at time t, and reward is represented with a subset of elements in the next current state vector (121) measured at t+1.

Another embodiment of the invention defines the optimal path as a component of a well-being improvement service. The calculation of optimal paths for individual users to achieve health and well-being goals is primarily conceived as a component of a well-being improvement service. As such, optimal path calculations tailor the specific behaviour change approaches and tools provided for an individual user over time, based on their personal characteristics and the overall behaviour change goal that they have set.

Another embodiment of the invention defines the optimal path as a service for third-parties. In addition, the calculation of optimal paths for individual users to achieve health and well-being goals may be offered as a service for third-party health and well-being improvement systems. Such an optimal path as a service would require defined inputs from the third-party, including personal characteristics of the service user, their health and well-being goal, and the range of approaches and tools available to help the user achieve their goal.

Note that in this text, the term "comprises" and its derivations (such as "comprising", etc.) should not be understood in an excluding sense, that is, these terms should not be interpreted as excluding the possibility that what is described and defined may include further elements, steps, etc.

## Claims

1. A computer-implemented method for optimizing behavioural changes of a user, **characterized by** comprising the following steps:
- setting a behavioural change goal for the user,
- for a specific time instant, t,
- adquiring static user characteristics (101) to generate a user characteristics vector (110),
- adquiring dynamic user characteristics which define a current user state (102) to generate a user current state vector (120),
- querying (103) an artificial intelligence system (400) using the generated user characteristics vector (110) and user current state vector (120) as input to generate as output (104) a behavioural change tool vector (130) for the specific time instant, t, the output (104) being delivered to the user who makes one or more selections to change behaviour based on the output (104);
- mapping the user characteristics vector (110), user current state vector (120) and the behavioural change tool vector (130) generated for the specific time instant, t, to a next current state vector (120) generated for a next time instant, t+1;
- repeating the querying (103) to the artificial intelligence system (400) using as input the three vectors, user characteristics vector, user current state vector and behavioural change tool vector, updated over time, until the mapping indicates that the behavioural change goal is achieved; the selections taken by the user defining an optimal path for behavioural changes.

2. The method according to claim 1, wherein the generation as output (104) of the behavioural change tool vector (130) by the artificial intelligence system (400) comprises reinforcement learning, wherein:
- a subset of elements from the current state vector (120) generated for the specific time instant, t, represents a state space;
- a subset of elements from the current state vector (120) and the user characteristics vector (110), both generated for the specific time instant, t, represents a context; and
- a subset of elements in the next current state vector (121) generated for the next time instant, t+1, represents a reward.

3. The method according to any preceeding claim, wherein the behavioural change tool vector (130) comprises a trigger for the user to take a selection from a set of actions, a content which is the set of actions, and an end which is a final action identifying the set behavioural change goal.

4. The method according to any preceeding claim, wherein the user current state vector (120) comprises three sub-vectors:
- a current behaviours sub-vector containing data referred to patterns of activities of daily living and of the behavioural change goal;
- a current dynamic personal characteristics sub-vector containing data referred to momentary cognitive processing;
- an emotional status sub-vector of emotional status containing data referred to assessment indices of user's emotional and well-being.

5. The method according to any preceeding claim, wherein the user current state vector (120) is generated with dynamic user characteristics acquired by using internal or external services carried out by user's terminal devices.

6. The method according to any preceeding claim, wherein the user current state vector (120) is generated with dynamic user characteristics acquired by ambient sensors.

7. The method according to any preceeding claim, wherein the user current state vector (120) is generated with dynamic user characteristics acquired by direct manual inputs from the user.

8. The method according to any preceeding claim, wherein the user characteristics vector (110) is generated with static user characteristics (101) comprising:
- demographic information (210),
- personal traits (220),
- preferences (230) including a wide range of personal descriptors,
- biological characteristics (240), and
- positive and negative conceptual associations (250), defining object associations with respect to the behavioural change goal.

9. The method according to any preceeding claim, wherein the user characteristics vector (110) is generated with static user characteristics (101) adquired by using external services carried out by user's terminal devices.

10. The method according to any preceeding claim, wherein the user characteristics vector (110) is generated with static user characteristics (101) adquired by external databases.

11. The method according to any preceeding claim, wherein the user characteristics vector (110) is generated with static user characteristics (101) adquired by ambient sensors.

12. The method according to any preceeding claim, wherein the user characteristics vector (110) is generated with static user characteristics (101) adquired by direct manual inputs from the user.

13. An artificial intelligence system (400) for optimizing behavioural changes of a user **characterized by** comprising processing means implementing artificial intelligence (410) and a database (420) on which the artificial intelligence (410) is built, and the artificial intelligence (410) being configured to, for a behavioural change goal set for the user:
- generate a user characteristics vector (110) for a specific time instant, t, using adquired static user characteristics (101);
- generate a user current state vector (120) for a specific time instant, t, using adquired dynamic user characteristics which define a current user state (102);
- generate a behavioural change tool vector (130) for the specific time instant, t, and deliver the behavioural change tool vector (130) as an output (104) from the artificial intelligence system (400) to the user, who makes one or more selections to change behaviour based on the behavioural change tool vector (130); the behavioural change tool vector (130) being generated using the generated user characteristics vector (110) and user current state vector (120) as inputs to the artificial intelligence system (400) in a query (103);
- map the user characteristics vector (110), user current state vector (120) and the behavioural change tool vector (130) generated for the specific time instant, t, to a next current state vector (120) generated for a next time instant, t+1;
- repeat the query (103) using as input the three vectors, user characteristics vector, user current state vector and behavioural change tool vector, updated over time, until the mapping indicates that the behavioural change goal is achieved; the selections taken by the user defining an optimal path for behavioural changes.

14. The system (400) according to claim 13, wherein the artificial intelligence (410) is configured to generate the behavioural change tool vector (130) by reinforcement learning, wherein:
- a subset of elements from the current state vector (120) generated for the specific time instant, t, represents a state space;
- a subset of elements from the current state vector (120) and the user characteristics vector (110), both generated for the specific time instant, t, represents a context; and
- a subset of elements in the next current state vector (121) generated for the next time instant, t+1, represents a reward.

15. The system (400) according to any of claims 13-14, wherein the artificial intelligence (410) is configured to generate the behavioural change tool vector (130) comprising a trigger for the user to take a selection from a set of actions, a content which is the set of actions, and an end which is a final action identifying the set behavioural change goal.

16. The system (400) according to any of claims 13-15, wherein the artificial intelligence (410) is configured to generate the user current state vector (120) comprising three sub-vectors:
- a current behaviours sub-vector containing data referred to patterns of activities of daily living and of the behavioural change goal;
- a current dynamic personal characteristics sub-vector containing data referred to momentary cognitive processing;
- an emotional status sub-vector of emotional status containing data referred to assessment indices of user's emotional and well-being.

17. The system (400) according to any of claims 13-16, wherein the artificial intelligence (410) is configured to generate the user current state vector (120) with dynamic user characteristics acquired by using internal or external services carried out by user's terminal devices.

18. The system (400) according to any of claims 13-17, wherein the artificial intelligence (410) is configured to generate the user current state vector (120) with dynamic user characteristics acquired by ambient sensors.

19. The system (400) according to any of claims 13-18, wherein the artificial intelligence (410) is configured to generate the user current state vector (120) with dynamic user characteristics acquired by direct manual inputs from the user.

20. The system (400) according to any of claims 13-19, wherein the artificial intelligence (410) is configured to generate the user characteristics vector (110) with static user characteristics (101) comprising:
- demographic information (210),
- personal traits (220),
- preferences (230) including a wide range of personal descriptors,
- biological characteristics (240), and
- positive and negative conceptual associations (250), defining object associations with respect to the behavioural change goal.

21. The system (400) according to any of claims 13-20, wherein the artificial intelligence (410) is configured to generate the user characteristics vector (110) with static user characteristics (101) adquired by using external services carried out by user's terminal devices.

22. The system (400) according to any of claims 13-21, wherein the artificial intelligence (410) is configured to generate the user characteristics vector (110) with static user characteristics (101) adquired by external databases.

23. The system (400) according to any of claims 13-22, wherein the artificial intelligence (410) is configured to generate the user characteristics vector (110) with static user characteristics (101) adquired by ambient sensors.

24. The system (400) according to any of claims 13-20, wherein the artificial intelligence (410) is configured to generate the user characteristics vector (110) with static user characteristics (101) adquired by direct manual inputs from the user.
